(19)

Europäisches
Patentamt
European
Patent Office
Office européen
des brevets

(11) **EP 3 719 103 A1**

(12) **EUROPEAN PATENT APPLICATION**
published in accordance with Art. 153(4) EPC

(43) Date of publication:
**07.10.2020 Bulletin 2020/41**

(21) Application number: **18883406.3**

(22) Date of filing: **15.10.2018**

(51) Int Cl.:
*C11D 1/14* *(2006.01)*        *A61K 8/46* *(2006.01)*
*A61Q 5/02* *(2006.01)*        *A61Q 19/10* *(2006.01)*
*C07C 303/42* *(2006.01)*      *C07C 309/20* *(2006.01)*

(86) International application number:
**PCT/JP2018/038284**

(87) International publication number:
**WO 2019/106982 (06.06.2019 Gazette 2019/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.11.2017 JP 2017228899**

(71) Applicant: **Kao Corporation
Chuo-Ku
Tokyo 103-8210 (JP)**

(72) Inventors:
• **SAWASE,Kanae
Wakayama-shi
Wakayama 640-8580 (JP)**

• **HORI,Hiroshi
Wakayama-shi
Wakayama 640-8580 (JP)**
• **KAYAHARA,Junpei
Wakayama-shi
Wakayama 640-8580 (JP)**
• **NOMURA,Masato
Wakayama-shi
Wakayama 640-8580 (JP)**

(74) Representative: **Hoffmann Eitle
Patent- und Rechtsanwälte PartmbB
Arabellastraße 30
81925 München (DE)**

(54) **INTERNAL OLEFIN SULFONATE COMPOSITION AND METHOD FOR PRESERVING INTERNAL OLEFIN SULFONATE**

(57)     Provided are an internal olefin sulfonate composition which shows excellent hue stability and excellent smell stability even when preserved for a long period of time, and a method for preserving an internal olefin sulfonate. The internal olefin sulfonate composition according to the present invention comprises an internal olefin sulfonate, one or more kinds of alkaline agents selected from among an alkali metal hydroxide and an alkaline earth metal hydroxide and water, wherein the content of the alkaline agents is 0.2 to 15 mmol inclusive per mol of the internal olefin sulfonate. The method according to the present invention for preserving an internal olefin sulfonate comprises mixing the internal olefin sulfonate at least with an alkaline agent to give a composition wherein the content of the alkaline agent is 0.2 to 15 mmol inclusive per mol of the internal olefin sulfonate.

**EP 3 719 103 A1**

**Description**

TECHNICAL FIELD

The present invention relates to an internal olefin sulfonate composition useful as a base for detergents, and a method for preserving an internal olefin sulfonate.

BACKGROUND ART

Conventionally, anionic surfactants, particularly, alkylsulfates and polyoxyalkylene alkyl ether sulfates are widely used as washing components for home use and industrial use since the surfactants are excellent in washing power and foaming power. As one of the anionic surfactants, a report has been made about an olefin sulfonate, particularly, an internal olefin sulfonate obtained using, as a raw material, an internal olefin, which has a double bond not at any terminal of the olefin but at an inside thereof.

The internal olefin sulfonate is generally obtained by reacting an internal olefin with a sulfur trioxide gas to sulfate the olefin, neutralizing the resultant sulfonated internal olefin, and further hydrolyzing the neutralized product. In recent years, characteristics such as good rinsing properties have been found in detergents compounded with an internal olefin sulfonate, and such a sulfonate can be expected to be used in various situations in the future.

For example, aiming at providing an aqueous hair detergent having excellent performances in all of foaming during washing, rinsing properties in rinsing, and gloss of hair after drying, Patent Document 1 proposes an aqueous hair detergent containing the following components (A), (B), and (C), and water and having a pH of 2 to 5 at 25°C when the hair detergent was diluted 20-fold by mass with water:

(A) a sulfonated internal olefin,
(B) a specific organic solvent, and
(C) at least one organic carboxylic acid selected from the group consisting of a hydroxymonocarboxylic acid and a dicarboxylic acid.

In addition, aiming at providing a sterilizing detergent composition that exhibits a high sterilizing effect even when used for a short time, and has excellent performances of foaming during washing, foam quality, and rinsing properties, Patent Document 2 proposes a sterilizing detergent composition, which contains:

component (a): 0.5% by mass or more and 20% by mass or less of an internal olefin sulfonate having 12 to 18 carbon atoms,
component (b): 0.1% by mass or more and 1.0% by mass or less of an oil-soluble sterilizing agent, and
component (c): 0.25% by mass or more and 7.0% by mass or less of a solvent having a LogP of 2.0 or more and 4.0 or less,
and having a pH at 25°C of 4.7 or more and 7.5 or less.

Further, aiming at providing a tableware detergent composition for hand washing where foam disappears suddenly during rinsing and rinsing is completed with a small amount of water while exhibiting rich foaming during washing and long lasting of the foam during washing, Patent Document 3 proposes a tableware detergent for hand washing containing 1% by mass to 50% by mass of the following component (a), 0.5% by mass to 5% by mass of the following component (b), and the following component (c), wherein the mass ratio of (c)/(a) is from 0.01 to 1:

component (a): an internal olefin sulfonate having 8 to 24 carbon atoms,
component (b): a fatty acid having 8 to 22 carbon atoms or salts thereof,
component (c): at least one compound selected from the following (c1), (c2), and (c3),
(c1): an alkyl or alkenyl alkanolamide having an alkyl or alkenyl group of 8 to 22 carbon atoms,
(c2): a fatty acid amidopropyl betaine having an alkyl or alkenyl group of 7 to 21 carbon atoms, and
(c3) : a polyoxyethylene alkyl or alkenyl amine having an alkyl or alkenyl group of 8 to 22 carbon atoms.

PRIOR ART DOCUMENTS

PATENT DOCUMENTS

Patent Document 1: JP-A-2014-76983
Patent Document 2: JP-A-2015-178548
Patent Document 3: JP-A-2016-147928

## SUMMARY OF THE INVENTION

### PROBLEMS TO BE SOLVED BY THE INVENTION

However, when a conventional composition containing an internal olefin sulfonate is preserved for a long period of time, its hue may deteriorate, or its smell may increase.

The present invention provides an internal olefin sulfonate composition having excellent hue stability and smell stability even when preserved for a long period of time, and a method for preserving an internal olefin sulfonate.

### MEANS FOR SOLVING THE PROBLEMS

When a composition containing an internal olefin sulfonate is preserved for a long period of time, the cause of deterioration in hue or occurrence of strong smell is not clear. This is considered due to oxidation, decomposition, bonding, polymerization, or the like of the internal olefin sulfonate in the composition during preservation. Then, the present inventors have found that an internal olefin sulfonate composition having excellent hue stability and smell stability can be obtained by controlling the pH of the composition containing the internal olefin sulfonate or by controlling the content of an alkaline agent in the composition containing the internal olefin sulfonate, even when preserved for a long period of time.

That is, the present invention relates to an internal olefin sulfonate composition comprising an internal olefin sulfonate and water, wherein

when a content of the internal olefin sulfonate is 10% by mass or less, pH at 25°C is 8.5 or more and 11.0 or less, or when a content of the internal olefin sulfonate is more than 10% by mass, pH at 25°C when the content of the internal olefin sulfonate is adjusted with water to 10% by mass is 8.5 or more and 11.0 or less.

Also, the present invention relates to an internal olefin sulfonate composition comprising an internal olefin sulfonate, one or more kinds of alkaline agents selected from among an alkali metal hydroxide and an alkaline earth metal hydroxide, and water, wherein

a content of the alkaline agent is 0.2 mmol or more and 15 mmol or less per 1 mol of the internal olefin sulfonate.

Also, the present invention relates to a method for preserving an internal olefin sulfonate in a composition comprising the internal olefin sulfonate and water, the method comprising:

when a content of the internal olefin sulfonate in the composition is 10% by mass or less, adjusting pH at 25°C of the composition to 8.5 or more and 11.0 or less, or

when a content of the internal olefin sulfonate in the composition is more than 10% by mass, adjusting pH at 25°C of a diluted solution to 8.5 or more and 11.0 or less, obtained by diluting the composition with water so that the content of the internal olefin sulfonate becomes 10% by mass.

Also, the present invention relates to a method for preserving an internal olefin sulfonate, comprising mixing an internal olefin sulfonate with at least one or more alkaline agents selected from among an alkali metal hydroxide and an alkaline earth metal hydroxide to form a composition,

wherein a content of the alkaline agent in the composition is 0.2 mmol or more and 15 mmol or less per 1 mol of the internal olefin sulfonate.

### EFFECT OF THE INVENTION

The internal olefin sulfonate composition of the present invention hardly changes hue and smell even when preserved for a long period of time and is excellent in stability thereof. Therefore, the internal olefin sulfonate composition of the present invention is useful as a base for various detergents. Further, according to the method for preserving an internal olefin sulfonate of the present invention, the quality of the internal olefin sulfonate can be stabilized for a long time.

### MODE FOR CARRYING OUT THE INVENTION

The internal olefin sulfonate composition of the present invention comprises an internal olefin sulfonate and water, in which when a content of the internal olefin sulfonate is 10% by mass or less, pH at 25°C is 8.5 or more and 11.0 or less, or when a content of the internal olefin sulfonate is more than 10% by mass, pH at 25°C when the content of the internal olefin sulfonate is adjusted with water to 10% by mass is 8.5 or more and 11.0 or less. When the content of the internal olefin sulfonate is more than 10% by mass, such a content is adjusted with water to 10% by mass. This is because the internal olefin sulfonate composition becomes a high viscosity as the concentration of the internal olefin sulfonate is increased, resulting in difficulty to measure the pH of the composition.

Further, the internal olefin sulfonate composition of the present invention comprises an internal olefin sulfonate, one or more kinds of alkaline agents selected from among an alkali metal hydroxide and an alkaline earth metal hydroxide, and water . A content of the alkaline agent is 0.2 mmol or more and 15 mmol or less per 1 mol of the internal olefin sulfonate.

As the internal olefin sulfonate, known ones can be used without any particular limitation. For example, the internal olefin sulfonate can be obtained by sulfonation, neutralization and hydrolysis of a raw material internal olefin (an olefin having a double bond inside the olefin chain). That is, when the internal olefin is sulfonated, β-sultone is quantitatively generated, and a part of the β-sultone is changed to γ-sultone and olefin sulfonic acid. Further, these compounds are converted into a hydroxyalkane sulfonate and an olefin sulfonate by neutralizing/hydrolyzing steps (for example, J. Am. Oil Chem. Soc. 69, 39 (1992)) . The term "internal olefin" has a broad meaning including a case where a so-called α-olefin having a double bond at the 1-position of the carbon chain is included in a small amount. Here, the hydroxy group of the obtained hydroxyalkane sulfonate is located inside the carbon chain, and the double bond of the olefin sulfonate is located inside the carbon chain. The obtained product is mainly a mixture thereof, which may include a case where a hydroxyalkane sulfonate having a hydroxy group at the terminal of the carbon chain or an olefin sulfonate having a double bond at the terminal of the carbon chain is partly contained in a small amount. In the present invention, these products and their mixtures are collectively referred to as internal olefin sulfonates. The hydroxyalkane sulfonate is also referred to as a hydroxy form of an internal olefin sulfonate (hereinafter, also referred to as HAS), and the olefin sulfonate is also referred to as an olefin form of an internal olefin sulfonate (hereinafter, also referred to as IOS).

The number of carbon atoms in the internal olefin sulfonate is preferably 10 or more, more preferably 12 or more, still more preferably 14 or more, even still more preferably 16 or more, and also preferably 24 or less, more preferably 22 or less, still more preferably 20 or less, even more preferably 18 or less, from the viewpoint of washing performance and the like. The internal olefin sulfonate may be used alone or in combination of two or more types thereof having different number of carbon atoms.

When the internal olefin sulfonate is a mixture containing a hydroxyalkane sulfonate and an olefin sulfonate, the mass ratio of the content of the hydroxyalkane sulfonate to the content of the olefin sulfonate (hydroxy form/olefin form) is preferably from 40/60 to 95/5, more preferably from 60/40 to 90/10, still more preferably from 70/30 to 88/12, and even still more preferably from 75/25 to 86/14, from the viewpoints of washing performance and foaming performance. Incidentally, after separating the hydroxyalkane sulfonate and the olefin sulfonate by HPLC, the mass ratio of the content of the hydroxyalkane sulfonate to the content of the olefin sulfonate can be measured by the method described in Examples.

The content of the internal olefin sulfonate having a sulfonic acid group at the 2-position is preferably 6.8% by mass or more, more preferably 8.6% by mass or more, still more preferably 11.3% by mass or more, even still more preferably 13.1% by mass or more, from the viewpoints of reducing production cost and improving productivity. Further, from the viewpoints of washing performance and foaming performance, the content of the internal olefin sulfonate having a sulfonic acid group at the 2-position is preferably 24.9% by mass or less, more preferably 24.0% by mass or less, still more preferably 23.1% by mass or less, even still more preferably 22.2% by mass or less, even still more preferably 21.3% by mass or less, even still more preferably 20.4% by mass or less, even still more preferably 19.5% by mass or less, even still more preferably 18.6% by mass or less. The content of the internal olefin sulfonate having a sulfonic acid group at the 2-position can be measured by gas chromatography.

The internal olefin sulfonate includes an olefin sulfonate having a double bond at the terminal of the carbon chain in an amount of preferably 0.01% by mass or more, more preferably 0.1% by mass or more, still more preferably 0.5% by mass or more, and even still more preferably 1.0% by mass or more, from the viewpoints of reducing production cost and improving productivity. Further, from the viewpoints of washing performance and foaming performance, the internal olefin sulfonate includes an olefin sulfonate having a double bond at the terminal of the carbon chain in an amount of preferably 10% by mass or less, more preferably 5.3% by mass or less, still more preferably 5.0% by mass or less, even still more preferably 4.7% by mass or less, even still more preferably 4.4% by mass or less, even still more preferably 4.1% by mass or less, even still more preferably 3.7% by mass or less, even still more preferably 3.4% by mass or less, and even still more preferably 3.1% by mass or less.

The internal olefin sulfonate can be produced by a known production method as described above, for example, by sulfonating, neutralizing, and hydrolyzing an internal olefin. Hereinafter, each step will be specifically described.

<Sulfonating Step>

The sulfonating step is a step of causing an internal olefin to react with sulfur trioxide to yield a sulfonated product.

In order to obtain the internal olefin sulfonate, the content of an internal olefin having a double bond at the 2-position is preferably 10% by mass or more, more preferably 13% by mass or more, still more preferably 18% by mass or more, even still more preferably 21% by mass or more, and preferably 42% by mass or less, more preferably 41% by mass or less, still more preferably 39% by mass or less, even still more preferably 37% by mass or less, even still more preferably 36% by mass or less, even still more preferably 34% by mass or less, even still more preferably 33% by mass or less, even still more preferably 31% by mass or less.

The internal olefin can be produced by a known method, for example, a method described in International Publication No. 2011/052732.

Sulfur trioxide is not particularly limited, but it is preferable to use sulfur trioxide gas from the viewpoint of improving reactivity.

From the viewpoint of reducing the amount of impurities, the reaction molar ratio of sulfur trioxide to the internal olefin is preferably 0.9 or more, more preferably 0.95 or more, and still more preferably 0.98 or more. From the viewpoints of reducing the amount of impurities and suppressing excessive reaction, the reaction molar ratio is preferably 1.1 or less, more preferably 1.05 or less, and still more preferably 1.03 or less.

<Neutralizing Step>

The neutralizing step is a step of reacting the obtained sulfonated product with an alkaline agent to obtain a neutralized product.

Examples of the alkaline agent used for neutralization include inorganic alkali compounds. Examples of the inorganic alkali compound include alkali metal hydroxides such as sodium hydroxide and potassium hydroxide; alkaline earth metal hydroxides such as magnesium hydroxide and calcium hydroxide; alkali metal carbonates such as sodium carbonate and potassium carbonate. These may be used alone or in combination of two or more types thereof.

The alkaline agent is preferably one or more kinds selected from among an alkali metal hydroxide and an alkaline earth metal hydroxide, more preferably an alkali metal hydroxide, and still more preferably sodium hydroxide or potassium hydroxide, from the viewpoints of availability and economy.

The alkaline agent is used in the form of a solid or an aqueous solution. From the viewpoints of handling and productivity, an aqueous solution of the alkaline agent is preferable.

The amount of the alkaline agent to be added is preferably 1.02 mole times or more, more preferably 1.03 mole times or more, still more preferably 1.04 mole times or more, and preferably 1.5 mole times or less, more preferably 1.3 mole times or less, still more preferably 1.1 mole times or less, based on a theoretical acid value of the sulfonated product.

The treatment temperature of the neutralizing step is preferably 0°C or higher, more preferably 10°C or higher, still more preferably 20°C or higher, from the viewpoint of improving reactivity, and preferably 60°C or lower, more preferably 40°C or lower, from the viewpoint of suppressing side reactions.

The treatment time of the neutralizing step is preferably 5 minutes or more, more preferably 15 minutes or more, still more preferably 30 minutes or more, from the viewpoint of completing the reaction, and preferably 5 hours or less, more preferably 2 hours or less, still more preferably 90 minutes or less, from the viewpoints of economy and the like.

<Hydrolyzing Step>

The hydrolyzing step is a step of hydrolyzing the resultant neutralized product.

In the hydrolyzing step, the temperature at the time of the hydrolysis is preferably 120°C or higher, more preferably 140°C or higher, even more preferably 160°C or higher to improve the reactivity. The temperature is also preferably 220°C or lower, more preferably 180°C or lower to restrain the decomposition of the product.

The treating period in the hydrolyzing step is preferably 30 minutes or longer, more preferably 45 minutes or longer to complete the reaction. The period is also preferably 4 hours or shorter, more preferably 3 hours or shorter, even more preferably 2 hours or shorter, even more preferably 90 minutes or shorter to improve the productivity.

An aqueous solution containing an internal olefin sulfonate is obtained by the hydrolyzing step. If necessary, treatments such as desalination, decolorization, and reduction may be performed. Further, an internal olefin sulfonate may be obtained from an aqueous solution containing an internal olefin sulfonate by performing an extraction operation or the like.

The internal olefin sulfonate composition of the present invention contains one or more kinds of alkaline agents selected from among an alkali metal hydroxide and an alkaline earth metal hydroxide. The alkaline agent is preferably an alkali metal hydroxide, more preferably sodium hydroxide or potassium hydroxide, from the viewpoints of availability and economy.

The content of one or more kinds of alkaline agents selected from among an alkali metal hydroxide and an alkaline earth metal hydroxide in the internal olefin sulfonate composition is 0.2 mmol or more, preferably 1.5 mmol or more, more preferably 2.5 mmol or more, still more preferably 5 mmol or more, and 15 mmol or less, preferably 10 mmol or less, per 1 mol of the internal olefin sulfonate, from the viewpoints of hue stability and smell stability during preservation.

If necessary, the content of the alkaline agent can be adjusted to a target range by adding an internal olefin sulfonate or an alkaline agent to the internal olefin sulfonate composition. The content of the alkaline agent in the internal olefin sulfonate composition can be determined by neutralization titration.

Further, when the content of the internal olefin sulfonate is 10% by mass or less, or when the content of the internal olefin sulfonate is adjusted with water to 10% by mass in case the content of the internal olefin sulfonate is more than 10% by mass, an alkaline agent is preferably contained in the internal olefin sulfonate composition of the present invention so that the pH at 25°C is adjusted to 8.5 to 11.0. The alkaline agent is not particularly limited and may be the above-mentioned alkaline agents or other alkaline agents.

The internal olefin sulfonate composition contains water (such as ion-exchanged water).

The internal olefin sulfonate composition of the present invention is a liquid (including a paste), and its production method is not particularly limited. For example, the internal olefin sulfonate composition may be produced using an aqueous solution containing the internal olefin sulfonate or may be produced by mixing the internal olefin sulfonate, water (such as ion-exchanged water) and the alkaline agent.

The content of the internal olefin sulfonate in the internal olefin sulfonate composition is preferably 10% by mass or more, more preferably 20% by mass or more, still more preferably 25% by mass or more, even still more preferably 30% by mass or more, even still more preferably more than 30% by mass, even still more preferably 32% by mass or more, even still more preferably 36% by mass or more, even still more preferably 40% by mass or more, from the viewpoint of hue stability and smell stability during preservation. From the viewpoint of handleability, the content of the internal olefin sulfonate in the internal olefin sulfonate composition is preferably 80% by mass or less, more preferably 70% by mass or less. When the content of the internal olefin sulfonate is out of the above range, water (such as ion-exchanged water) may be mixed into the composition or the composition may be concentrated to adjust the content of the internal olefin sulfonate. The content of the internal olefin sulfonate in the internal olefin sulfonate composition can be determined by a streaming potential method.

The content of water (such as ion-exchanged water) in the internal olefin sulfonate composition is preferably an amount remaining as a residue other than the above components, and is preferably 17% by mass or more, more preferably 27% by mass or more, from the viewpoint of handleability, and preferably 89% by mass or less, more preferably 79% by mass or less, still more preferably 74% by mass or less, even still more preferably 68% by mass or less, even still more preferably 58% by mass or less, from the viewpoints of hue stability and smell stability during preservation.

When the content of the internal olefin sulfonate in the composition is 10% by mass or less, or when the content of the internal olefin sulfonate is adjusted with water (such as ion-exchanged water) to 10% by mass in case the content of the internal olefin sulfonate in the composition is more than 10% by mass, the pH at 25°C is preferably 8.5 or more, more preferably 9.0 or more, still more preferably 9.4 or more, even still more preferably 9.8 or more, and preferably 11.0 or less, more preferably 10.5 or less, from the viewpoints of hue stability and smell stability during preservation. The pH can be adjusted, for example, by adding the alkaline agent and the following buffers or acids.

The internal olefin sulfonate composition preferably contains a buffer from the viewpoints of maintaining the hue stability and smell stability during preservation and the pH within the above range.

The buffer is not particularly limited, and examples thereof include carbonates such as sodium carbonate and potassium carbonate; hydrogen carbonates such as sodium hydrogen carbonate and potassium hydrogen carbonate; borates such as sodium borate and potassium borate; silicates such as sodium silicate and potassium silicate . These may be used alone or in combination of two or more types thereof. Among these, from the viewpoints of availability and economy, preferred are carbonates, and more preferred is sodium carbonate.

From the viewpoint of maintaining the pH within the above range, the content of the buffer is preferably 0.01 parts by mass or more, more preferably 0.05 parts by mass or more, still more preferably 0.1 parts by mass or more, per 100 parts by mass of the internal olefin sulfonate. In addition, the content of the buffer is preferably 3.0 parts by mass or less, more preferably 2.5 parts by mass or less, still more preferably 2.0 parts by mass or less, even still more preferably 1.0 part by mass or less, even still more preferably 0.5 parts by mass or less, from the viewpoint of reducing the amount of impurities.

It is not necessary to add a stabilizer such as an acid to the internal olefin sulfonate composition, but an acid may be added to adjust the content of the alkaline agent so that the pH is adjusted to the above range. The acid is present as an acid salt in the composition.

The acid is not particularly limited, and examples thereof include inorganic acids such as sulfuric acid, hydrochloric acid, phosphoric acid, and boric acid; and organic acids such as citric acid, malic acid, maleic acid, fumaric acid, and succinic acid. These may be used alone or in combination of two or more types thereof. Of these, preferred are citric acid, phosphoric acid, and boric acid, and more preferred is citric acid.

The amount of the acid to be added may be appropriately adjusted so that the pH falls within the above range. The amount of the acid is preferably 10 parts by mass or less, more preferably 1 part by mass or less, still more preferably 0.1 parts by mass or less, and even still more preferably 0.01 parts by mass or less, per 100 parts by mass of the internal olefin sulfonate.

The internal olefin sulfonate composition may contain an anionic surfactant or a nonionic surfactant from the viewpoint of storage stability or viscosity adjustment.

Examples of the anionic surfactant include a sulfuric acid ester salt having 10 to 22 carbon atoms, such as an alkyl sulfate having 10 to 22 carbon atoms, an alkenyl sulfate having 10 to 22 carbon atoms, a polyoxyalkylene alkyl ether sulfate having 10 to 22 carbon atoms, a polyoxyalkylene alkenyl ether sulfate having 10 to 22 carbon atoms, and a polyoxyalkylene alkyl phenyl ether sulfate having 10 to 22 carbon atoms; a sulfonate having 10 to 22 carbon atoms, such as a sulfosuccinic acid alkyl ester salt having 10 to 22 carbon atoms, a polyoxyalkylene sulfosuccinic acid alkyl ester salt having 10 to 22 carbon atoms, an alkane sulfonate having 10 to 22 carbon atoms, an acyl isethionate having 10 to 22 carbon atoms, an acylmethyl taurate having 10 to 22 carbon atoms, and an alkyl sulfoacetate having 10 to 22 carbon atoms; a carboxylic acid salt having 10 to 22 carbon atoms, such as a fatty acid salt having 10 to 22 carbon atoms, a polyoxyalkylene alkyl ether carboxylate having 10 to 22 carbon atoms, a polyoxyalkylene alkenyl ether carboxylate having 10 to 22 carbon atoms, and a polyoxyalkylene alkyl phenyl ether carboxylate having 10 to 22 carbon atoms; a phosphoric acid ester salt having 10 to 22 carbon atoms, such as an alkyl phosphate having 10 to 22 carbon atoms and a polyoxyalkylene alkyl ether phosphate having 10 to 22 carbon atoms; an amino acid salt having an acyl group of 10 to 22 carbon atoms, such as an acyl glutamic acid salt, an acyl alanine salt, an acyl glycine salt, an acyl sarcosine salt, and an acyl arginine salt. These may be used alone or in combination of two or more kinds thereof.

Examples of the nonionic surfactant include polyalkylene glycol-type nonionic surfactants such as polyoxyalkylene alkyl ether having 10 to 22 carbon atoms, polyoxyethylene sorbitan fatty acid ester, polyoxyethylene sorbitol fatty acid ester, polyoxyethylene glycerin fatty acid ester, polyoxyethylene fatty acid ester, polyoxyethylene alkyl phenyl ether having 10 to 22 carbon atoms, and polyoxyalkylene (hardened) castor oil having 10 to 22 carbon atoms; polyhydric alcohol-type nonionic surfactants such as sucrose fatty acid ester, polyglycerin alkyl ether having 10 to 22 carbon atoms, polyglycerin fatty acid ester, alkylglycosides having 10 to 22 carbon atoms, and acylated alkylglucamides; and fatty acid alkanolamide. The fatty acid or acyl group of the nonionic surfactant is a fatty acid having 10 to 22 carbon atoms. Specific examples include fatty acid monoalkanolamides such as coconut oil fatty acid monoethanolamide and coconut oil fatty acid N-methyl monoethanolamide. These may be used alone or in combination of two or more kinds thereof.

The internal olefin sulfonate composition may contain a hydrotrope in order to improve storage stability.

Examples of the hydrotrope include toluene sulfonic acid, xylene sulfonic acid, cumene sulfonic acid, and salts thereof (such as sodium salt, potassium salt, magnesium salt, and amine salt) . These may be used alone or in combination of two or more kinds thereof.

The internal olefin sulfonate composition may contain a solvent from the viewpoint of storage stability or viscosity adjustment.

Examples of the solvent include ethanol, isopropyl alcohol, ethylene glycol, propylene glycol, diethylene glycol, dipropylene glycol, glycerin, isoprene glycol, propylene glycol monomethyl ether, propylene glycol monoethyl ether, 3-methyl-3-methoxybutanol, phenoxyethanol, phenyl glycol, phenoxy isopropanol, butyl diglycol (diethylene glycol monobutyl ether), dibutylene diglycol, and benzyl alcohol. These may be used alone or in combination of two or more kinds thereof.

Before and after preservation at 50°C for 20 days, the internal olefin sulfonate composition has a hue change rate of preferably 5% or less, more preferably 3% or less, and still more preferably 2% or less.

Further, as described above, the method for preserving an internal olefin sulfonate according to the present invention comprises: in a composition comprising an internal olefin sulfonate and water, when a content of the internal olefin sulfonate in the composition is 10% by mass or less, adjusting pH at 25°C of the composition to 8.5 or more and 11.0 or less, or when a content of the internal olefin sulfonate in the composition is more than 10% by mass, adjusting pH at 25°C of a diluted solution to 8.5 or more and 11.0 or less, obtained by diluting the composition with water so that the content of the internal olefin sulfonate becomes 10% by mass. By adjusting the pH to 8.5 or more and 11.0 or less, it is possible to improve the preservation stability of the internal olefin sulfonate. When the content of the internal olefin sulfonate is more than 10% by mass, the internal olefin sulfonate is diluted to 10% by mass with water for the reason that when the concentration of the internal olefin sulfonate is high, the viscosity of the internal olefin sulfonate composition becomes high, resulting in difficulty to measure the pH of the composition.

In addition, the method for preserving an internal olefin sulfonate of the present invention comprises, as described above, mixing an internal olefin sulfonate with at least one or more alkaline agent selected from among an alkali metal hydroxide and an alkaline earth metal hydroxide to form a composition, in which a content of the alkaline agent is 0.2 mmol or more and 15 mmol or less per 1 mol of the internal olefin sulfonate. By mixing the internal olefin sulfonate with the alkaline agent to form a composition in which the content of the alkaline agent is adjusted to the above range, the preservation stability of the internal olefin sulfonate can be improved.

Each component in the composition and the content thereof, and the pH of the composition are the same as described above, and thus description thereof is omitted.

The internal olefin sulfonate composition of the present invention can be used as a base compounded in hard surface detergents such as dishwashing detergent and residential detergent, clothing detergent, clothing treatment agent, clothing softener, body detergent, and hair detergent, and also can be used for the production of these.

The dishwashing detergent of the present invention is used, for example, for washing plastics, glass, pottery, stainless steel, wood, sponges, cutting boards, countertops, tables, and chairs. Such dishwashing detergent is suitably used for washing hard surfaces where coloring, smell, etc. pose a problem, preferably used for washing plastics, glass, ceramics, sponges, cutting boards, countertops, tables, and chairs.

The residential detergent of the present invention is used for washing plastics, glass, pottery, stainless steel, wood, paper, leather, rubber, walls, flooring, bathtubs, washbasins, toilet bowls, windows, and the like, and is suitably used for cleaning hard surfaces where coloring, smell etc. pose a problem, preferably used for cleaning plastics, glass, pottery, paper, walls, flooring, bathtubs, toilet bowls, and the like.

The hard surface detergents such as dishwashing detergents and residential detergents, clothing detergents, clothing treatment agents, clothing softeners, body detergents, and hair detergents may contain a known component together with the internal olefin sulfonate composition of the present invention, for example, a surfactant other than the internal olefin sulfonate, and an auxiliary agent, etc.

Examples of the auxiliaries include foam enhancers, defoamers, polymers, oils, hydrophilic solvents, silicones, humectants, preservatives, viscosity modifiers, preservatives, antibacterial agents, anti-dandruff agents, anti-inflammatory agents, anti-oxidants, ultraviolet absorbers, sequestering agents, pearlescent agents, pigments, fragrances, enzymes, bleaching agents, bleaching activators, chelating agents, water-soluble salts, polyvalent cations, and pH adjusters. The auxiliary agent is used alone or in combination of two or more types thereof depending on the use.

The content of the surfactant (including the internal olefin sulfonate) in the hard surface detergent, residential detergent or dishwashing detergent is not particularly limited, but is preferably 0.5% by mass or more, more preferably 15% by mass or more, and preferably 60% by mass or less, more preferably 30% by mass or less.

The hard surface detergent, residential detergent or dishwashing detergent preferably contains a pH adjuster (such as citric acid). The content of the pH adjuster in the hard surface detergent, residential detergent or dishwashing detergent is not particularly limited, but is preferably 0.001% by mass or more, and preferably 8% by mass or less, more preferably 5% by mass or less.

The present invention discloses, besides the above, the following embodiments.

<1> An internal olefin sulfonate composition comprising an internal olefin sulfonate and water, wherein when a content of the internal olefin sulfonate is 10% by mass or less, pH at 25°C is 8.5 or more and 11.0 or less, or when a content of the internal olefin sulfonate is more than 10% by mass, pH at 25°C when the content of the internal olefin sulfonate is adjusted with water to 10% by mass is 8.5 or more and 11.0 or less.

<2> An internal olefin sulfonate composition comprising an internal olefin sulfonate, one or more kinds of alkaline agents selected from among an alkali metal hydroxide and an alkaline earth metal hydroxide, and water, wherein a content of the alkaline agent is 0.2 mmol or more and 15 mmol or less per 1 mol of the internal olefin sulfonate.

<3> The internal olefin sulfonate composition according to <1> or <2>, wherein the number of carbon atoms in the internal olefin sulfonate is preferably 10 or more, more preferably 12 or more, still more preferably 14 or more, even still more preferably 16 or more, and preferably 24 or less, more preferably 22 or less, still more preferably 20 or less, even more preferably 18 or less.

<4> The internal olefin sulfonate composition according to any one of <1> to <3>, wherein when the internal olefin sulfonate is a mixture containing a hydroxyalkane sulfonate and an olefin sulfonate, the mass ratio of the content of the hydroxyalkane sulfonate to the content of the olefin sulfonate (hydroxy form/olefin form) is preferably from 40/60 to 95/5, more preferably from 60/40 to 90/10, still more preferably from 70/30 to 88/12, and even still more preferably from 75/25 to 86/14.

<5> The internal olefin sulfonate composition according to any one of <1> to <4>, wherein the content of the internal olefin sulfonate having a sulfonic acid group at the 2-position is preferably 6.8% by mass or more, more preferably 8.6% by mass or more, still more preferably 11.3% by mass or more, even still more preferably 13.1% by mass or more, and preferably 24.9% by mass or less, more preferably 24.0% by mass or less, still more preferably 23.1% by mass or less, even still more preferably 22.2% by mass or less, even still more preferably 21.3% by mass or less, even still more preferably 20.4% by mass or less, even still more preferably 19.5% by mass or less, even still more preferably 18.6% by mass or less.

<6> The internal olefin sulfonate composition according to any one of <1> to <5>, wherein the internal olefin sulfonate includes an olefin sulfonate having a double bond at the terminal of the carbon chain in an amount of preferably 0.01% by mass or more, more preferably 0.1% by mass or more, still more preferably 0.5% by mass or more, and even still more preferably 1.0% by mass or more, and preferably 10% by mass or less, more preferably 5.3% by mass or less, still more preferably 5.0% by mass or less, even still more preferably 4.7% by mass or less, even still more preferably 4.4% by mass or less, even still more preferably 4.1% by mass or less, even still more preferably 3.7% by mass or less, even still more preferably 3.4% by mass or less, and even still more preferably 3.1% by mass or less.

<7> The internal olefin sulfonate composition according to any one of <2> to <6>, wherein the alkaline agent is preferably an alkali metal hydroxide, and more preferably sodium hydroxide or potassium hydroxide.

<8> The internal olefin sulfonate composition according to any one of <2> to <7>, wherein the content of one or more kinds of alkaline agents selected from among an alkali metal hydroxide and an alkaline earth metal hydroxide in the internal olefin sulfonate composition is preferably 1.5 mmol or more, more preferably 2.5 mmol or more, still more preferably 5 mmol or more, and preferably 10 mmol or less, per 1 mol of the internal olefin sulfonate.

<9> The internal olefin sulfonate composition according to any one of <1> to <8>, wherein the content of the internal olefin sulfonate in the internal olefin sulfonate composition is preferably 10% by mass or more, more preferably 18% by mass or more, still more preferably 20% by mass or more, even still more preferably 25% by mass or more, even still more preferably 30% by mass or more, even still more preferably more than 30% by mass, even still more preferably 32% by mass or more, even still more preferably 36% by mass or more, even still more preferably 40% by mass or more, and preferably 80% by mass or less, more preferably 70% by mass or less.

<10> The internal olefin sulfonate composition according to any one of <2> to <7>, wherein

preferably, the content of one or more kinds of alkaline agents selected from among an alkali metal hydroxide and an alkaline earth metal hydroxide in the internal olefin sulfonate composition is 1.5 mmol or more and 10 mmol or less, per 1 mol of the internal olefin sulfonate, and the content of the internal olefin sulfonate in the internal olefin sulfonate composition is 10% by mass or more and 80% by mass or less,

more preferably, the content of one or more kinds of alkaline agents selected from among an alkali metal hydroxide and an alkaline earth metal hydroxide in the internal olefin sulfonate composition is 2.5 mmol or more and 10 mmol or less, per 1 mol of the internal olefin sulfonate, and the content of the internal olefin sulfonate in the internal olefin sulfonate composition is 20% by mass or more and 70% by mass or less,

still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, and the content of the internal olefin sulfonate in the internal olefin sulfonate composition is 25% by mass or more and 70% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, and the content of the internal olefin sulfonate in the internal olefin sulfonate composition is 30% by mass or more and 70% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, and the content of the internal olefin sulfonate in the internal olefin sulfonate composition is more than 30% by mass and 70% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, and the content of the internal olefin sulfonate in the internal olefin sulfonate composition is 32% by mass or more and 70% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, and the content of the internal olefin sulfonate in the internal olefin sulfonate composition is 36% by mass or more and 70% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, and the content of the internal olefin sulfonate in the internal olefin sulfonate composition is 40% by mass or more and 70% by mass or less.

<11> The internal olefin sulfonate composition according to any one of <1> to <10>, wherein the content of water in the internal olefin sulfonate composition is preferably 17% by mass or more, more preferably 27% by mass or more, and preferably 89% by mass or less, more preferably 79% by mass or less, still more preferably 74% by mass or less, even still more preferably 68% by mass or less, even still more preferably 58% by mass or less.

<12> The internal olefin sulfonate composition according to any one of <1> to <8>, wherein

preferably, the content of the internal olefin sulfonate in the internal olefin sulfonate composition is 10% by mass or more and 80% by mass or less, and the content of water in the internal olefin sulfonate composition is 17% by mass or more and 89% by mass or less,

more preferably, the content of the internal olefin sulfonate in the internal olefin sulfonate composition is 20% by mass or more and 70% by mass or less, and the content of water in the internal olefin sulfonate composition is 27% by mass or more and 79% by mass or less,

still more preferably, the content of the internal olefin sulfonate in the internal olefin sulfonate composition is 25% by mass or more and 70% by mass or less, and the content of water in the internal olefin sulfonate composition is 27% by mass or more and 74% by mass or less,

even still more preferably, the content of the internal olefin sulfonate in the internal olefin sulfonate composition is 30% by mass or more and 70% by mass or less, and the content of water in the internal olefin sulfonate composition is 27% by mass or more and 68% by mass or less,

even still more preferably, the content of the internal olefin sulfonate in the internal olefin sulfonate composition is more than 30% by mass and 70% by mass or less, and the content of water in the internal olefin sulfonate composition is 27% by mass or more and 58% by mass or less,

even still more preferably, the content of the internal olefin sulfonate in the internal olefin sulfonate composition is 32% by mass or more and 70% by mass or less, and the content of water in the internal olefin sulfonate composition is 27% by mass or more and 58% by mass or less,

even still more preferably, the content of the internal olefin sulfonate in the internal olefin sulfonate composition is 36% by mass or more and 70% by mass or less, and the content of water in the internal olefin sulfonate composition is 27% by mass or more and 58% by mass or less,

even still more preferably, the content of the internal olefin sulfonate in the internal olefin sulfonate composition is 40% by mass or more and 70% by mass or less, and the content of water in the internal olefin sulfonate composition is 27% by mass or more and 58% by mass or less.

<13> The internal olefin sulfonate composition according to any one of <2> to <7>, wherein

preferably, the content of one or more kinds of alkaline agents selected from among an alkali metal hydroxide and an alkaline earth metal hydroxide in the internal olefin sulfonate composition is 1.5 mmol or more and 10 mmol or less, per 1 mol of the internal olefin sulfonate, the content of the internal olefin sulfonate in the internal olefin sulfonate composition is 10% by mass or more and 80% by mass or less, and the content of water in the internal olefin sulfonate composition is 17% by mass or more and 89% by mass or less,

more preferably, the content of one or more kinds of alkaline agents selected from among an alkali metal hydroxide and an alkaline earth metal hydroxide in the internal olefin sulfonate composition is 2.5 mmol or more and 10 mmol or less, per 1 mol of the internal olefin sulfonate, the content of the internal olefin sulfonate in the internal olefin sulfonate composition is 20% by mass or more and 70% by mass or less, and the content of water in the internal olefin sulfonate composition is 27% by mass or more and 79% by mass or less,

still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 25% by mass or more and 70% by mass or less, and the content of water in the internal olefin sulfonate composition is 27% by mass or more and 74% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 30% by mass or more and 70% by mass or less, and the content of water in the internal olefin sulfonate composition is 27% by mass or more and 68% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is more than 30% by mass and 70% by mass or less, and the content of water in the internal olefin sulfonate composition is 27% by mass or more and 58% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 32% by mass or more and 70% by mass or less, and the content of water in the internal olefin sulfonate composition is 27% by mass or more and 58% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 36% by mass or more and 70% by mass or less, and the content of water in the internal olefin sulfonate composition is 27% by mass or more and 58% by mass or less,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 40% by mass or more and 70% by mass or less, and the content of water in the internal olefin sulfonate composition is 27% by mass or more and 58% by mass or less.

<14> The internal olefin sulfonate composition according to any one of <12> to <13>, wherein when the content of the internal olefin sulfonate in the composition is 10% by mass or less, or when the content of the internal olefin sulfonate is adjusted with water to 10% by mass in case the content of the internal olefin sulfonate in the composition is more than 10% by mass, the pH at 25°C is preferably 8.5 or more, more preferably 9.0 or more, still more preferably 9.4 or more, even still more preferably 9.8 or more, and preferably 11.0 or less, more preferably 10.5 or less.

<15> The internal olefin sulfonate composition according to any one of <1> to <14>, further comprising a buffer.

<16> The internal olefin sulfonate composition according to <15>, wherein the buffer is preferably carbonates, more preferably sodium carbonate.

<17> The internal olefin sulfonate composition according to <15> or <16>, wherein the content of the buffer is preferably 0.01 parts by mass or more, more preferably 0.05 parts by mass or more, still more preferably 0.1 parts by mass or more, and preferably 3.0 parts by mass or less, more preferably 2.5 parts by mass or less, still more preferably 2.0 parts by mass or less, even still more preferably 1.0 part by mass or less, even still more preferably 0.5 parts by mass or less, per 100 parts by mass of the internal olefin sulfonate.

<18> The internal olefin sulfonate composition according to any one of <2> to <7>, wherein

preferably, the content of one or more kinds of alkaline agents selected from among an alkali metal hydroxide and an alkaline earth metal hydroxide in the internal olefin sulfonate composition is 1.5 mmol or more and 10 mmol or less, per 1 mol of the internal olefin sulfonate, the content of the internal olefin sulfonate in the internal olefin sulfonate composition is 10% by mass or more and 80% by mass or less, further comprising a buffer, and the content of the buffer is preferably 0.01 parts by mass or more and 3.0 parts by mass or less, per 100 parts by mass of the internal olefin sulfonate,

more preferably, the content of one or more kinds of alkaline agents selected from among an alkali metal hydroxide and an alkaline earth metal hydroxide in the internal olefin sulfonate composition is 2.5 mmol or more and 10 mmol or less, per 1 mol of the internal olefin sulfonate, the content of the internal olefin sulfonate in the internal olefin sulfonate composition is 20% by mass or more and 70% by mass or less, further comprising a buffer, and the content of the buffer is preferably 0.05 parts by mass or more and 2.5 parts by mass or less, per 100 parts by mass of the internal olefin sulfonate,

still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 25% by mass or more and 70% by mass or less, further comprising a buffer, and the content of the buffer is preferably 0.1 parts by mass or more and 2.0 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 30% by mass or more and 70% by mass or less, further comprising a buffer, and the content of the buffer is preferably 0.1 parts by mass or more and 1.0 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is more than 30% by mass and 70% by mass or less, further comprising a buffer, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 32% by mass or more and 70% by mass or less, further comprising a buffer, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 36% by mass or more and 70% by mass or less, further comprising a buffer, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 40% by mass or more and 70% by mass or less, further comprising a buffer, and the content of the buffer is preferably 0.1 parts by mass or more and 0.5 parts by mass or less, per 100 parts by mass of the internal olefin sulfonate.

<19> The internal olefin sulfonate composition according to any one of <1> to <8>, wherein

preferably, the content of the internal olefin sulfonate in the internal olefin sulfonate composition is 10% by mass or more and 80% by mass or less, the content of water in the internal olefin sulfonate composition is 17% by mass or more and 89% by mass or less, further comprising a buffer, and the content of the buffer is preferably 0.01 parts by mass or more and 3.0 parts by mass or less, per 100 parts by mass of the internal olefin sulfonate,

more preferably, the content of the internal olefin sulfonate in the internal olefin sulfonate composition is 20% by mass or more and 70% by mass or less, the content of water in the internal olefin sulfonate composition is 27% by mass or more and 79% by mass or less, further comprising a buffer, and the content of the buffer is preferably 0.05 parts by mass or more and 2.5 parts by mass or less, per 100 parts by mass of the internal olefin sulfonate,

still more preferably, the content of the internal olefin sulfonate in the internal olefin sulfonate composition is 25% by mass or more and 70% by mass or less, the content of water in the internal olefin sulfonate composition is 27% by mass or more and 74% by mass or less, further comprising a buffer, and the content of the buffer is preferably 0.1 parts by mass or more and 2.0 parts by mass or less, per 100 parts by mass of the internal olefin sulfonate,

even still more preferably, the content of the internal olefin sulfonate in the internal olefin sulfonate composition is 30% by mass or more and 70% by mass or less, the content of water in the internal olefin sulfonate composition is 27% by mass or more and 68% by mass or less, further comprising a buffer, and the content of the buffer is preferably 0.1 parts by mass or more and 1.0 parts by mass or less, per 100 parts by mass of the internal olefin sulfonate,

even still more preferably, the content of the internal olefin sulfonate in the internal olefin sulfonate composition is more than 30% by mass and 70% by mass or less, the content of water in the internal olefin sulfonate composition is 27% by mass or more and 58% by mass or less, further comprising a buffer, and the content of the buffer is preferably 0.1 parts by mass or more and 0.5 parts by mass or less, per 100 parts by mass of the internal olefin sulfonate,

even still more preferably, the content of the internal olefin sulfonate in the internal olefin sulfonate composition is 32% by mass or more and 70% by mass or less, the content of water in the internal olefin sulfonate composition is 27% by mass or more and 58% by mass or less, further comprising a buffer, and the content of the buffer is preferably 0.1 parts by mass or more and 0.5 parts by mass or less, per 100 parts by mass of the internal olefin sulfonate,

even still more preferably, the content of the internal olefin sulfonate in the internal olefin sulfonate composition is 36% by mass or more and 70% by mass or less, the content of water in the internal olefin sulfonate composition is 27% by mass or more and 58% by mass or less, further comprising a buffer, and the content of the buffer is preferably 0.1 parts by mass or more and 0.5 parts by mass or less, per 100 parts by mass of the internal olefin sulfonate,

even still more preferably, the content of the internal olefin sulfonate in the internal olefin sulfonate composition is 40% by mass or more and 70% by mass or less, the content of water in the internal olefin sulfonate composition is 27% by mass or more and 58% by mass or less, further comprising a buffer, and the content of the buffer is preferably 0.1 parts by mass or more and 0.5 parts by mass or less, per 100 parts by mass of the internal olefin sulfonate.

<20> The internal olefin sulfonate composition according to any one of <2> to <7>, wherein

preferably, the content of one or more kinds of alkaline agents selected from among an alkali metal hydroxide and an alkaline earth metal hydroxide in the internal olefin sulfonate composition is 1.5 mmol or more and 10 mmol or less, per 1 mol of the internal olefin sulfonate, the content of the internal olefin sulfonate in the internal olefin sulfonate composition is 10% by mass or more and 80% by mass or less, the content of water in the internal olefin sulfonate composition is 17% by mass or more and 89% by mass or less, further comprising a buffer, and the content of the buffer is preferably 0.01 parts by mass or more and 3.0 parts by mass or less, per 100 parts by mass of the internal olefin sulfonate,

more preferably, the content of one or more kinds of alkaline agents selected from among an alkali metal hydroxide and an alkaline earth metal hydroxide in the internal olefin sulfonate composition is 2.5 mmol or more and 10 mmol or less, per 1 mol of the internal olefin sulfonate, the content of the internal olefin sulfonate in the internal olefin sulfonate composition is 20% by mass or more and 70% by mass or less, the content of water in the internal olefin sulfonate composition is 27% by mass or more and 79% by mass or less, further comprising a buffer, and the content of the buffer is preferably 0.05 parts by mass or more and 2.5 parts by mass or less, per 100 parts by mass of the internal olefin sulfonate,

still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 25% by mass or more and 70% by mass or less, the content of water in the internal olefin sulfonate composition is 27% by mass or more and 74% by mass or less, further comprising a buffer, and the content of the buffer is preferably 0.1 parts by mass or more and 2.0 parts by mass or less, per 100 parts by mass of the internal olefin sulfonate,
even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 30% by mass or more and 70% by mass or less, the content of water in the internal olefin sulfonate composition is 27% by mass or more and 68% by mass or less, further comprising a buffer, and the content of the buffer is preferably 0.1 parts by mass or more and 1.0 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is more than 30% by mass and 70% by mass or less, the content of water in the internal olefin sulfonate composition is 27% by mass or more and 58% by mass or less, further comprising a buffer, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 32% by mass or more and 70% by mass or less, the content of water in the internal olefin sulfonate composition is 27% by mass or more and 58% by mass or less, further comprising a buffer, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 36% by mass or more and 70% by mass or less, the content of water in the internal olefin sulfonate composition is 27% by mass or more and 58% by mass or less, further comprising a buffer, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the internal olefin sulfonate composition is 5 mmol or more and 10 mmol or less, per 1 mol of the internal olefin sulfonate, the content of the internal olefin sulfonate in the internal olefin sulfonate composition is 40% by mass or more and 70% by mass or less, the content of water in the internal olefin sulfonate composition is 27% by mass or more and 58% by mass or less, further comprising a buffer, and the content of the buffer is preferably 0.1 parts by mass or more and 0.5 parts by mass or less, per 100 parts by mass of the internal olefin sulfonate.

<21> The internal olefin sulfonate composition according to any one of <1> to <20>, wherein an acid is further added.
<22> The internal olefin sulfonate composition according to <21>, wherein the acid is preferably citric acid, phosphoric acid and boric acid, more preferably citric acid.
<23> The internal olefin sulfonate composition according to <21> or <22>, wherein the amount of the acid to be added is preferably 10 parts by mass or less, more preferably 1 part by mass or less, still more preferably 0.1 parts by mass or less, and even still more preferably 0.01 parts by mass or less, per 100 parts by mass of the internal olefin sulfonate.
<24> The internal olefin sulfonate composition according to any one of <1> to <23>, wherein further comprising one or more kinds of surfactants selected from among an anionic surfactant and a nonionic surfactant.
<25> The internal olefin sulfonate composition according to any one of <1> to <24>, wherein further comprising a hydrotrope.
<26> The internal olefin sulfonate composition according to any one of <1> to <25>, wherein further comprising a solvent.
<27> The internal olefin sulfonate composition according to any one of <1> to <26>, wherein before and after preservation at 50°C for 20 days, the internal olefin sulfonate composition has a hue change rate of preferably 5% or less, more preferably 3% or less, and still more preferably 2% or less.
<28> A method for preserving an internal olefin sulfonate in a composition comprising the internal olefin sulfonate and water, the method comprising:

when a content of the internal olefin sulfonate in the composition is 10% by mass or less, adjusting pH at 25°C of the composition to 8.5 or more and 11.0 or less, or
when a content of the internal olefin sulfonate in the composition is more than 10% by mass, adjusting pH at 25°C of a diluted solution to 8.5 or more and 11.0 or less, obtained by diluting the composition with water so that the content of the internal olefin sulfonate becomes 10% by mass.

<29> A method for preserving an internal olefin sulfonate, comprising mixing an internal olefin sulfonate with at least one or more alkaline agents selected from among an alkali metal hydroxide and an alkaline earth metal hydroxide to form a composition,
wherein a content of the alkaline agent in the composition is 0.2 mmol or more and 15 mmol or less per 1 mol of the internal olefin sulfonate.

<30> The method for preserving an internal olefin sulfonate according to <28> or <29>, wherein the number of carbon atoms in the internal olefin sulfonate is preferably 10 or more, more preferably 12 or more, still more preferably 14 or more, even still more preferably 16 or more, and preferably 24 or less, more preferably 22 or less, still more preferably 20 or less, even more preferably 18 or less.

<31> The method for preserving an internal olefin sulfonate according to any one of <28> to <30>, wherein when the internal olefin sulfonate is a mixture containing a hydroxyalkane sulfonate and an olefin sulfonate, the mass ratio of the content of the hydroxyalkane sulfonate to the content of the olefin sulfonate (hydroxy form/olefin form) is preferably from 40/60 to 95/5, more preferably from 60/40 to 90/10, still more preferably from 70/30 to 88/12, and even still more preferably from 75/25 to 86/14.

<32> The method for preserving an internal olefin sulfonate according to any one of <28> to <31>, wherein the content of the internal olefin sulfonate having a sulfonic acid group at the 2-position is preferably 6.8% by mass or more, more preferably 8.6% by mass or more, still more preferably 11.3% by mass or more, even still more preferably 13.1% by mass or more, and preferably 24.9% by mass or less, more preferably 24.0% by mass or less, still more preferably 23.1% by mass or less, even still more preferably 22.2% by mass or less, even still more preferably 21.3% by mass or less, even still more preferably 20.4% by mass or less, even still more preferably 19.5% by mass or less, even still more preferably 18.6% by mass or less.

<33> The method for preserving an internal olefin sulfonate according to any one of <28> to <32>, wherein the internal olefin sulfonate includes an olefin sulfonate having a double bond at the terminal of the carbon chain in an amount of preferably 0.01% by mass or more, more preferably 0.1% by mass or more, still more preferably 0.5% by mass or more, and even still more preferably 1.0% by mass or more, and preferably 10% by mass or less, more preferably 5.3% by mass or less, still more preferably 5.0% by mass or less, even still more preferably 4.7% by mass or less, even still more preferably 4.4% by mass or less, even still more preferably 4.1% by mass or less, even still more preferably 3.7% by mass or less, even still more preferably 3.4% by mass or less, and even still more preferably 3.1% by mass or less.

<34> The method for preserving an internal olefin sulfonate according to any one of <29> to <33>, wherein the alkaline agent is preferably an alkali metal hydroxide, and more preferably sodium hydroxide or potassium hydroxide.

<35> The method for preserving an internal olefin sulfonate according to any one of <29> to <34>, wherein the content of one or more kinds of alkaline agents selected from among an alkali metal hydroxide and an alkaline earth metal hydroxide in the composition is preferably 1.5 mmol or more, more preferably 2.5 mmol or more, still more preferably 5 mmol or more, and preferably 9.8 mmol or less, per 1 mol of the internal olefin sulfonate.

<36> The method for preserving an internal olefin sulfonate according to any one of <28> to <35>, wherein the content of the internal olefin sulfonate in the internal olefin sulfonate composition is preferably 10% by mass or more, more preferably 18% by mass or more, still more preferably 20% by mass or more, even still more preferably 25% by mass or more, even still more preferably 30% by mass or more, even still more preferably more than 30% by mass, even still more preferably 32% by mass or more, even still more preferably 36% by mass or more, even still more preferably 40% by mass or more, and preferably 80% by mass or less, more preferably 70% by mass or less.

<37> The method for preserving an internal olefin sulfonate according to any one of <29> to <34>, wherein preferably, the content of one or more kinds of alkaline agents selected from among an alkali metal hydroxide and an alkaline earth metal hydroxide in the composition is 1.5 mmol or more and 10 mmol or less, per 1 mol of the internal olefin sulfonate, and the content of the internal olefin sulfonate in the internal olefin sulfonate composition is 10% by mass or more and 80% by mass or less,
more preferably, the content of one or more kinds of alkaline agents selected from among an alkali metal hydroxide and an alkaline earth metal hydroxide in the composition is 2.5 mmol or more and 10 mmol or less, per 1 mol of the internal olefin sulfonate, and the content of the internal olefin sulfonate in the internal olefin sulfonate composition is 20% by mass or more and 70% by mass or less,
still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, and the content of the internal olefin sulfonate in the internal olefin sulfonate composition is 25% by mass or more and 70% by mass or less,
even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, and the content of the internal olefin sulfonate in the internal olefin sulfonate composition is 30% by mass or more and 70% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, and the content of the internal olefin sulfonate in the internal olefin sulfonate composition is more than 30% by mass and 70% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, and the content of the internal olefin sulfonate in the internal olefin sulfonate composition is 32% by mass or more and 70% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, and the content of the internal olefin sulfonate in the internal olefin sulfonate composition is 36% by mass or more and 70% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, and the content of the internal olefin sulfonate in the internal olefin sulfonate composition is 40% by mass or more and 70% by mass or less,

<38> The method for preserving an internal olefin sulfonate according to any one of <29> to <37>, wherein water is further mixed in the composition, and the content of water in the composition is preferably 17% by mass or more, more preferably 27% by mass or more, and preferably 89% by mass or less, more preferably 79% by mass or less, still more preferably 74% by mass or less, even still more preferably 68% by mass or less, even still more preferably 58% by mass or less.

<39> The method for preserving an internal olefin sulfonate according to any one of <29> to <35>, wherein preferably, the content of the internal olefin sulfonate in the composition is 10% by mass or more and 80% by mass or less, water is further mixed in the composition, and the content of water in the composition is 17% by mass or more and 89% by mass or less,

more preferably, the content of the internal olefin sulfonate in the composition is 20% by mass or more and 70% by mass or less, water is further mixed in the composition, and the content of water in the composition is 27% by mass or more and 79% by mass or less,

still more preferably, the content of the internal olefin sulfonate in the composition is 25% by mass or more and 70% by mass or less, water is further mixed in the composition, and the content of water in the composition is 27% by mass or more and 74% by mass or less,

even still more preferably, the content of the internal olefin sulfonate in the composition is 30% by mass or more and 70% by mass or less, water is further mixed in the composition, and the content of water in the composition is 27% by mass or more and 68% by mass or less,

even still more preferably, the content of the internal olefin sulfonate in the composition is more than 30% by mass and 70% by mass or less, water is further mixed in the composition, and the content of water in the composition is 27% by mass or more and 58% by mass or less,

even still more preferably, the content of the internal olefin sulfonate in the composition is 32% by mass or more and 70% by mass or less, water is further mixed in the composition, and the content of water in the composition is 27% by mass or more and 58% by mass or less,

even still more preferably, the content of the internal olefin sulfonate in the composition is 36% by mass or more and 70% by mass or less, water is further mixed in the composition, and the content of water in the composition is 27% by mass or more and 58% by mass or less,

even still more preferably, the content of the internal olefin sulfonate in the composition is 40% by mass or more and 70% by mass or less, water is further mixed in the composition, and the content of water in the composition is 27% by mass or more and 58% by mass or less.

<40> The method for preserving an internal olefin sulfonate according to any one of <29> to <34>, wherein preferably, the content of one or more kinds of alkaline agents selected from among an alkali metal hydroxide and an alkaline earth metal hydroxide in the composition is 1.5 mmol or more and 10 mmol or less, per 1 mol of the internal olefin sulfonate, the content of the internal olefin sulfonate in the composition is 10% by mass or more and 80% by mass or less, water is further mixed in the composition, and the content of water in the composition is 17% by mass or more and 89% by mass or less,

more preferably, the content of one or more kinds of alkaline agents selected from among an alkali metal hydroxide and an alkaline earth metal hydroxide in the composition is 2.5 mmol or more and 10 mmol or less, per 1 mol of the internal olefin sulfonate, the content of the internal olefin sulfonate in the composition is 20% by mass or more and 70% by mass or less, water is further mixed in the composition, and the content of water in the composition is 27% by mass or more and 79% by mass or less,

still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 25% by mass or more and 70% by mass or less, water is further mixed in the composition, and the content of water in the composition is 27% by mass or more and 74% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 30% by mass or more and 70% by mass or less, water is further mixed in the composition, and the content of water in the composition is 27% by mass or more and 68% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is more than 30% by mass and 70% by mass or less, water is further mixed in the composition, and the content of water in the composition is 27% by mass or more and 58% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 32% by mass or more and 70% by mass or less, water is further mixed in the composition, and the content of water in the composition is 27% by mass or more and 58% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 36% by mass or more and 70% by mass or less, water is further mixed in the composition, and the content of water in the composition is 27% by mass or more and 58% by mass or less,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 40% by mass or more and 70% by mass or less, water is further mixed in the composition, and the content of water in the composition is 27% by mass or more and 58% by mass or less.

<41> The method for preserving an internal olefin sulfonate according to any one of <29> to <40>, wherein when the content of the internal olefin sulfonate in the composition is 10% by mass or less, or when the content of the internal olefin sulfonate is adjusted with water (such as ion-exchanged water) to 10% by mass in case the content of the internal olefin sulfonate in the composition is more than 10% by mass, the pH at 25°C is preferably 8.5 or more, more preferably 9.0 or more, still more preferably 9.4 or more, even still more preferably 9.8 or more, and preferably 11.0 or less, more preferably 10.5 or less.

<42> The method for preserving an internal olefin sulfonate according to any one of <28> to <41>, wherein a buffer is further added in the composition.

<43> The method for preserving an internal olefin sulfonate according to <42>, wherein the buffer is preferably carbonates, more preferably sodium carbonate.

<44> The method for preserving an internal olefin sulfonate according to <42> or <43>, wherein the content of the buffer in the composition is preferably 0.01 parts by mass or more, more preferably 0.05 parts by mass or more, still more preferably 0.1 parts by mass or more, and preferably 3.0 parts by mass or less, more preferably 2.5 parts by mass or less, still more preferably 2.0 parts by mass or less, even still more preferably 1.0 part by mass or less, even still more preferably 0.5 parts by mass or less, per 100 parts by mass of the internal olefin sulfonate.

<45> The method for preserving an internal olefin sulfonate according to any one of <29> to <34>, wherein preferably, the content of one or more kinds of alkaline agents selected from among an alkali metal hydroxide and an alkaline earth metal hydroxide in the composition is 1.5 mmol or more and 10 mmol or less, per 1 mol of the internal olefin sulfonate, the content of the internal olefin sulfonate in the composition is 10% by mass or more and 80% by mass or less, a buffer is further added in the composition, and the content of the buffer is preferably 0.01 parts by mass or more and 3.0 parts by mass or less, per 100 parts by mass of the internal olefin sulfonate,

more preferably, the content of one or more kinds of alkaline agents selected from among an alkali metal hydroxide and an alkaline earth metal hydroxide in the composition is 2.5 mmol or more and 10 mmol or less, per 1 mol of the internal olefin sulfonate, the content of the internal olefin sulfonate in the composition is 20% by mass or more and 70% by mass or less, a buffer is further added in the composition, and the content of the buffer is preferably 0.05 parts by mass or more and 2.5 parts by mass or less, per 100 parts by mass of the internal olefin sulfonate,

still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 25% by mass or more and 70% by mass or less, a buffer is further added in the composition, and the content of the buffer is preferably 0.1 parts by mass or more and 2.0 parts by mass or less, per 100 parts by mass of the internal olefin sulfonate, even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 30% by mass or more and 70% by mass or less, a buffer is further added in the composition, and the content of the buffer is preferably 0.1 parts by mass or more and 1.0 parts by mass or less, per 100 parts by mass of the internal olefin sulfonate, even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is more than 30% by mass and 70% by mass or less, a buffer is further added in the composition, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 32% by mass or more and 70% by mass or less, a buffer is further added in the composition, and the content of the buffer is preferably 0.1 parts by mass or more and 0.5 parts by mass or less, per 100 parts by mass of the internal olefin sulfonate, even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 36% by mass or more and 70% by mass or less, a buffer is further added in the composition, and the content of the buffer is preferably 0.1 parts by mass or more and 0.5 parts by mass or less, per 100 parts by mass of the internal olefin sulfonate, even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 40% by mass or more and 70% by mass or less, a buffer is further added in the composition, and the content of the buffer is preferably 0.1 parts by mass or more and 0.5 parts by mass or less, per 100 parts by mass of the internal olefin sulfonate.

<46> The method for preserving an internal olefin sulfonate according to any one of <29> to <35>, wherein preferably, the content of the internal olefin sulfonate in the composition is 10% by mass or more and 80% by mass or less, water is further added in the composition, the content of water in the composition is 17% by mass or more and 89% by mass or less, a buffer is further added in the composition, and the content of the buffer in the composition is preferably 0.01 parts by mass or more and 3.0 parts by mass or less, per 100 parts by mass of the internal olefin sulfonate,

more preferably, the content of the internal olefin sulfonate in the composition is 20% by mass or more and 70% by mass or less, water is further added in the composition, the content of water in the composition is 27% by mass or more and 79% by mass or less, a buffer is further added in the composition, and the content of the buffer in the composition is preferably 0.05 parts by mass or more and 2.5 parts by mass or less, per 100 parts by mass of the internal olefin sulfonate,

still more preferably, the content of the internal olefin sulfonate in the composition is 25% by mass or more and 70% by mass or less, water is further added in the composition, the content of water in the composition is 27% by mass or more and 74% by mass or less, a buffer is further added in the composition, and the content of the buffer in the composition is preferably 0.1 parts by mass or more and 2.0 parts by mass or less, per 100 parts by mass of the internal olefin sulfonate,

even still more preferably, the content of the internal olefin sulfonate in the composition is 30% by mass or more and 70% by mass or less, water is further added in the composition, the content of water in the composition is 27% by mass or more and 68% by mass or less, a buffer is further added in the composition, and the content of the buffer in the composition is preferably 0.1 parts by mass or more and 1.0 parts by mass or less, per 100 parts by mass of the internal olefin sulfonate,

even still more preferably, the content of the internal olefin sulfonate in the composition is more than 30% by mass and 70% by mass or less, water is further added in the composition, the content of water in the composition is 27% by mass or more and 58% by mass or less, a buffer is further added in the composition, and the content of the buffer in the composition is preferably 0.1 parts by mass or more and 0.5 parts by mass or less, per 100 parts by mass of the internal olefin sulfonate,

even still more preferably, the content of the internal olefin sulfonate in the composition is 32% by mass or more and 70% by mass or less, water is further added in the composition, the content of water in the composition is 27% by mass or more and 58% by mass or less, a buffer is further added in the composition, and the content of the buffer in the composition is preferably 0.1 parts by mass or more and 0.5 parts by mass or less, per 100 parts by mass of the internal olefin sulfonate,

even still more preferably, the content of the internal olefin sulfonate in the composition is 36% by mass or more and 70% by mass or less, water is further added in the composition, the content of water in the composition is 27% by mass or more and 58% by mass or less, a buffer is further added in the composition, and the content of the buffer in the composition is preferably 0.1 parts by mass or more and 0.5 parts by mass or less, per 100 parts by mass of the internal olefin sulfonate,

even still more preferably, the content of the internal olefin sulfonate in the composition is 40% by mass or more and 70% by mass or less, water is further added in the composition, the content of water in the composition is 27% by mass or more and 58% by mass or less, a buffer is further added in the composition, and the content of the buffer in the composition is preferably 0.1 parts by mass or more and 0.5 parts by mass or less, per 100 parts by mass of the internal olefin sulfonate.

<47> The method for preserving an internal olefin sulfonate according to any one of <29> to <34>, wherein preferably, the content of one or more kinds of alkaline agents selected from among an alkali metal hydroxide and an alkaline earth metal hydroxide in the composition is 1.5 mmol or more and 10 mmol or less, per 1 mol of the internal olefin sulfonate, the content of the internal olefin sulfonate in the composition is 10% by mass or more and 80% by mass or less, water is further added in the composition, the content of water in the composition is 17% by mass or more and 89% by mass or less, a buffer is further added in the composition, and the content of the buffer is preferably 0.01 parts by mass or more and 3.0 parts by mass or less, per 100 parts by mass of the internal olefin sulfonate,

more preferably, the content of one or more kinds of alkaline agents selected from among an alkali metal hydroxide and an alkaline earth metal hydroxide in the composition is 2.5 mmol or more and 10 mmol or less, per 1 mol of the internal olefin sulfonate, the content of the internal olefin sulfonate in the composition is 20% by mass or more and 70% by mass or less, water is further added in the composition, the content of water in the composition is 27% by mass or more and 79% by mass or less, a buffer is further added in the composition, and the content of the buffer is preferably 0.05 parts by mass or more and 2.5 parts by mass or less, per 100 parts by mass of the internal olefin sulfonate,

still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 25% by mass or more and 70% by mass or less, water is further added in the composition, the content of water in the composition is 27% by mass or more and 74% by mass or less, a buffer is further added in the composition, and the content of the buffer is preferably 0.1 parts by mass or more and 2.0 parts by mass or less, per 100 parts by mass of the internal olefin sulfonate,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 30% by mass or more and 70% by mass or less, water is further added in the composition, the content of water in the composition is 27% by mass or more and 68% by mass or less, a buffer is further added in the composition, and the content of the buffer is preferably 0.1 parts by mass or more and 1.0 parts by mass or less, per 100 parts by mass of the internal olefin sulfonate,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is more than 30% by mass and 70% by mass or less, water is further added in the composition, the content of water in the composition is 27% by mass or more and 58% by mass or less, a buffer is further added in the composition, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 32% by mass or more and 70% by mass or less, water is further added in the composition, the content of water in the composition is 27% by mass or more and 58% by mass or less, a buffer is further added in the composition, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 36% by mass or more and 70% by mass or less, water is further added in the composition, the content of water in the composition is 27% by mass or more and 58% by mass or less, a buffer is further added in the composition, and the content of the buffer is preferably 0.1 parts by mass or more and 0.5 parts by mass or less, per 100 parts by mass of the internal olefin sulfonate,

even still more preferably, the content of one or more kinds of alkaline agents selected from among an alkali metal hydroxide and an alkaline earth metal hydroxide in the composition is 5 mmol or more and 10 mmol or less, per 1 mol of the internal olefin sulfonate, the content of the internal olefin sulfonate in the composition is 40% by mass or more and 70% by mass or less, water is further added in the composition, the content of water in the composition is 27% by mass or more and 58% by mass or less, a buffer is further added in the composition, and the content of the buffer is preferably 0.1 parts by mass or more and 0.5 parts by mass or less, per 100 parts by mass of the internal olefin sulfonate.

<48> The method for preserving an internal olefin sulfonate according to any one of <28> to <47>, wherein an acid is further added in the composition.

<49> The method for preserving an internal olefin sulfonate according to <48>, wherein the acid is preferably citric acid, phosphoric acid and boric acid, more preferably citric acid.

<50> The method for preserving an internal olefin sulfonate according to <48> or <49>, wherein the amount of the acid to be added is preferably 10 parts by mass or less, more preferably 1 part by mass or less, still more preferably 0.1 parts by mass or less, and even still more preferably 0.01 parts by mass or less, per 100 parts by mass of the internal olefin sulfonate.

<51> The method for preserving an internal olefin sulfonate according to any one of <28> to <50>, wherein one or more kinds of surfactants selected from among an anionic surfactant and a nonionic surfactant is further added in the composition.

<52> The method for preserving an internal olefin sulfonate according to any one of <28> to <51>, wherein a hydrotrope is further added in the composition.

<53> The method for preserving an internal olefin sulfonate according to any one of <28> to <52>, wherein a solvent is further added in the composition.

<54> A hard surface detergent, a residential detergent or a dishwashing detergent comprising the internal olefin sulfonate composition according to any one of <1> to <27>.

<55> A hard surface detergent, a residential detergent or a dishwashing detergent added the internal olefin sulfonate composition according to any one of <1> to <27>.

<56> The hard surface detergent, the residential detergent or the dishwashing detergent according to <54> or <55> further comprising a surfactant other than the internal olefin sulfonate, and at least one or more auxiliary agents selected from among a fragrance, a preservative, an antibacterial agent, a pH adjuster (citric acid and the like) and a polyvalent cation.

<57> The hard surface detergent, the residential detergent or the dishwashing detergent according to <56>, wherein the content of the surfactant (including the internal olefin sulfonate) in the hard surface detergent, residential detergent or dishwashing detergent is preferably 0.5% by mass or more, more preferably 15% by mass or more, and preferably 60% by mass or less, more preferably 30% by mass or less.

<58> The hard surface detergent, the residential detergent or the dishwashing detergent according to <56>, wherein the content of the pH adjuster (citric acid and the like) in the hard surface detergent, residential detergent or dishwashing detergent is preferably 0.001% by mass or more, and preferably 8% by mass or less, more preferably 5% by mass or less.

<59> A clothing detergent comprising the internal olefin sulfonate composition according to any one of <1> to <27>.

<60> A clothing detergent added the internal olefin sulfonate composition according to any one of <1> to <27>.

<61> The clothing detergent according to <59> or <60> further comprising an enzyme.

<62> A clothing treatment agent comprising the internal olefin sulfonate composition according to any one of <1> to <27>.

<63> A clothing treatment agent added the internal olefin sulfonate composition according to any one of <1> to <27>.

<64> The clothing treatment agent according to <62> or <63> further comprising at least one or more auxiliary agents selected from among a hydrophilic solvent and a defoamer.

<65> A clothing softener comprising the internal olefin sulfonate composition according to any one of <1> to <27>.

<66> A clothing softener added the internal olefin sulfonate composition according to any one of <1> to <27>.

<67> The clothing softener according to <65> or <66> further comprising a silicone.

<68> A body detergent comprising the internal olefin sulfonate composition according to any one of <1> to <27>.

<69> A body detergent added the internal olefin sulfonate composition according to any one of <1> to <27>.

<70> A hair detergent comprising the internal olefin sulfonate composition according to any one of <1> to <27>.

<71> A hair detergent added the internal olefin sulfonate composition according to any one of <1> to <27>.

<72> Use of the internal olefin sulfonate composition according to any one of <1> to <27> for production of a hard surface detergent, a residential detergent or a dishwashing detergent.

<73> Use of the internal olefin sulfonate composition according to any one of <1> to <27> for production of a clothing detergent.

<74> Use of the internal olefin sulfonate composition according to any one of <1> to <27> for production of a clothing treatment agent.

<75> Use of the internal olefin sulfonate composition according to any one of <1> to <27> for production of a clothing softener.

<76> Use of the internal olefin sulfonate composition according to any one of <1> to <27> for production of a body detergent.

<77> Use of the internal olefin sulfonate composition according to any one of <1> to <27> for production of a hair detergent.

<78> The hard surface detergent or the dishwashing detergent according to any one of <54> to <58> used for washing plastics, glass, pottery, stainless steel, wood, sponges, cutting boards, countertops, tables, or chairs, preferably used for washing hard surfaces where coloring, smell, etc. pose a problem, more preferably used for washing plastics, glass, ceramics, sponges, cutting boards, countertops, tables, or chairs.

<79> Use of the internal olefin sulfonate composition for production of the hard surface detergent or the dishwashing detergent according to <72> used for washing plastics, glass, pottery, stainless steel, wood, sponges, cutting boards, countertops, tables, or chairs, preferably used for washing hard surfaces where coloring, smell, etc. pose a problem, more preferably used for washing plastics, glass, ceramics, sponges, cutting boards, countertops, tables, or chairs.

<80> The hard surface detergent or the residential detergent according to any one of <54> to <58> used for washing plastics, glass, pottery, stainless steel, wood, paper, leather, rubber, walls, flooring, bathtubs, washbasins, toilet bowls, or windows, preferably used for cleaning hard surfaces where coloring, smell etc. pose a problem, more preferably used for cleaning plastics, glass, pottery, paper, walls, flooring, bathtubs, or toilet bowls.

<81> Use of the internal olefin sulfonate composition for production of the hard surface detergent or the residential detergent according to <72> used for washing plastics, glass, pottery, stainless steel, wood, paper, leather, rubber, walls, flooring, bathtubs, washbasins, toilet bowls, or windows, preferably used for cleaning hard surfaces where coloring, smell etc. pose a problem, more preferably used for cleaning plastics, glass, pottery, paper, walls, flooring, bathtubs, or toilet bowls.

EXAMPLES

Hereinafter, the present invention will be specifically described based on Examples. In the following Examples and Comparative Examples, "%" means "% by mass" unless otherwise specified. The methods for measuring various physical properties are as follows.

<Method for measuring double bond position of internal olefin>

Double bond positions of internal olefin were measured by gas chromatography (hereinafter abbreviated to GC). Specifically, dimethyl disulfide was caused to react with internal olefin to prepare dithionated derivatives thereof. Subsequently, individual components of the resultant derivatives were separated from each other by GC. From the individual peak areas, double bond positions of internal olefin were analyzed. Instruments and analyzing conditions used for the measurement were as follows: GC instrument "HP6890" (manufactured by Hewlett Packard Company), a column "Ultra-Alloy-1HT capillary column" (manufactured by Frontier Laboratories Ltd.; 30 m $\times$ 250 $\mu$m $\times$ 0.15 $\mu$m), and a detector (hydrogen flame ionization detector (FID)); and an injection temperature of 300°C, a detector temperature of 350°C, and a He flow rate of 4.6 mL/min.

<Method for measuring content of internal olefin sulfonate having sulfonic acid group at 2-position>

The binding position of the sulfonic acid group was measured by GC. Specifically, trimethylsilyldiazomethane was reacted with the internal olefin sulfonate to form a methyl esterified derivative, and then each component was separated by GC. Using the respective peak area ratios as mass ratios, the content of the internal olefin sulfonate having a sulfonic acid group at the 2-position was calculated. The apparatus and analysis conditions used for the measurement are as follows.

GC instrument: Agilent Technology 6850 (manufactured by Agilent Technologies Inc.)
Column: HP-1 capillary column, 30 m $\times$ 320 $\mu$m $\times$ 0.25 $\mu$m (manufactured by Agilent Technologies Inc.)

Detector: Hydrogen flame ionization detector (FID)
Injection temperature: 300°C
Detector temperature: 300°C
Helium flow rate: 1.0 mL/min
Oven: 60°C (0 min) → 10°C/min → 300°C (10 min)

<Method for measuring mass ratio of hydroxy form/olefin form>

The mass ratio of hydroxy form/olefin form of the internal olefin sulfonate was measured by HPLC-MS. Specifically, the hydroxy form and the olefin form were separated by HPLC, and each was identified by subjecting to MS. As a result, each ratio was determined from the HPLC-MS peak area. In addition, the apparatus and conditions used for the measurement are as follows.

HPLC system: Agilent Technology 1100 (manufactured by Agilent Technologies Inc.)
Column: L-column ODS 4.6 × 150 mm (manufactured by Chemicals Evaluation and Research Institutes, Japan)
Sample preparation: 1000-fold dilution with methanol
Eluent A: 10 mM ammonium acetate added water
Eluent B: 10 mM ammonium acetate added methanol
Gradient: 0 min (A/B = 30/70%) → 10 min (30/70%) → 55 min (0/100%) → 65 min (0/100%) → 66 min (30/70%) → 75 min (30/70%)
MS apparatus: Agilent Technology 1100 MS SL (G1946D)
MS detection: Anion detection m/z 60-1600, UV 240 nm

<Composition analysis>

The content of the internal olefin sulfonate in the internal olefin sulfonate compositions prepared in Examples and Comparative Examples was determined by the following streaming potential method. The content of potassium hydroxide or sodium hydroxide was determined by the following neutralization titration with HCl. The contents of sodium carbonate and citric acid were calculated from the amounts added.

<Streaming potential method>

An appropriate amount of a sample was weighed in a volumetric flask (about 1 g with an accuracy of three digits after the decimal point), and ion-exchanged water was added to make a total volume of 200 ml. The titration of a solution obtained by further diluting 10 ml of this sample solution with ion-exchanged water was performed using a potentiometric titrator (an automatic potentiometric titrator, manufactured by Kyoto Electrics Manufacturing Co., Ltd.). Using a 0.004 mol/L benzethonium chloride solution as a titrant, the titer when the potential of the sample solution became zero was read. The content of the internal olefin sulfonate in the sample was determined by the following equation.

$$\text{Content (\%) of internal olefin sulfonate} = \{(0.004 \times f \times b \times \text{molecular weight})/(a \times 10/200 \times 1000)\} \times 100$$

a: Sampling amount (g)
b: Benzethonium chloride solution used (ml)
f: Factor of benzethonium chloride solution

<Neutralization titration>

An appropriate amount (about 5 g with a precision of three digits after the decimal point) of a sample was weighed out and diluted with ion-exchanged water, and then about 0.05 g of a 1% phenolphthalein ethanol solution was added to the diluted solution to develop a red color. The sample solution was titrated with 1/10 N hydrochloric acid, and the titer when the color no longer developed red was read. The content of potassium hydroxide or sodium hydroxide in the sample was determined by the following equation.

Content of potassium hydroxide or sodium hydroxide (%)

= {(1/10 × f × d × M)/(c × 1000)} × 100

c: Sampling amount (g)
d: Amount of 1/10 N hydrochloric acid used (ml)
f: Factor of 1/10 N hydrochloric acid
M: Molecular weight of sodium hydroxide or potassium hydroxide

In addition, regarding a region (pH of less than 8.3) in which phenolphthalein does not color, the content of potassium hydroxide or sodium hydroxide in the sample was determined by the following method. When the content of potassium hydroxide or sodium hydroxide in the sample is plotted on the abscissa and the pH is plotted on the ordinate, the content of potassium hydroxide or sodium hydroxide in the sample was calculated from the measured pH value according to the following equation established by considering these relationships from pH 7 to 8.3 as approximately linear relationships.

Content of potassium hydroxide or sodium hydroxide (%)

= ((Measured pH) − 7)/1010.1

<Method for measuring pH when internal olefin sulfonate is adjusted with water to 10% by mass>

Measurement was performed using a pH meter (a pH meter D-51, manufactured by Horiba, Ltd.) calibrated with a pH standard solution. An internal olefin sulfonate composition was diluted with water (ion-exchanged water) so that the content of the internal olefin sulfonate became 10% by mass, and the obtained diluted solution was adjusted to 25°C. Then, a pH glass electrode of the pH meter was immersed in the diluted solution to read the display when stabilized.

<Method for measuring pH when content of internal olefin sulfonate is 10% by mass or less>

Measurement was performed using a pH meter (a pH meter D-51, manufactured by Horiba, Ltd.) calibrated with a pH standard solution. After adjusting an internal olefin sulfonate composition to 25°C, a pH glass electrode of the pH meter was immersed in the composition to read the display when stabilized.

<Method for measuring buffer content>

The content of a buffer (such as sodium carbonate) in an internal olefin sulfonate composition can be measured by ion chromatography. The following apparatus and conditions can be used for the measurement.

Ion chromatography equipment: Dionex ICS-2100, Dionex AS-AP (manufactured by Thermo Fisher Scientific K.K.)
Column: Dionex IonPac AS11-HC (manufactured by Thermo Fisher Scientific K.K.)
Eluent: EGC III KOH (manufactured by Thermo Fisher Scientific K.K.)
Suppressor: Dionex AERS 500 (manufactured by Thermo Fisher Scientific K.K.)

<Evaluation of preservation stability>

An internal olefin sulfonate composition was left to stand at 50°C for 20 days in a constant temperature bath in which the temperature in the bath could be controlled constant. The hue and smell were measured according to the following methods immediately after production and 20 days after standing.

(1) Hue

An internal olefin sulfonate composition was diluted with water (ion-exchanged water) so that the content of the internal olefin sulfonate became 10% by mass, thereby to obtain a diluted solution (50 mL). Thereafter, the diluted solution was adjusted to pH 7.0 ±0.1 using phosphoric acid to obtain a sample solution. Then, the sample solution was placed in a 10 mm glass cell, and the absorbance at a wavelength of 420 nm was measured with a spectrophotometer using water (ion-exchanged water) as a control. The value obtained by multiplying the obtained measured value by 1000 was defined as a 10% klett number. A hue change rate (%) when the value immediately after production was A and the value 20 days after standing was B was calculated by the following equation. From the viewpoint of hue stability, it is preferable that the absolute value of the hue change rate is small.

$$\text{Hue change rate (\%)} = 100 \times (B - A)/A$$

(2) Smell

The smell level of the internal olefin sulfonate composition immediately after production was set to 1, and the degree of change in the smell level of the internal olefin sulfonate composition after 20 days of standing was evaluated in comparison with the smell level of 1. The smell levels are classified into the following five levels, and a larger value means a stronger smell. The smell evaluation was performed by six researchers, and the average value of each evaluation result was rounded off and represented by one digit. The smell level after 20 days of standing is preferably 1, but levels 2 and 3 are also acceptable.

Level 1: Smell is equal to or weaker than that immediately after production of the internal olefin sulfonate composition.
Level 2: Smell is slightly stronger than that immediately after production of the internal olefin sulfonate composition.
Level 3: Smell is somewhat stronger than that immediately after production of the internal olefin sulfonate composition.
Level 4: Smell is stronger than that immediately after production of the internal olefin sulfonate composition.
Level 5: Smell is very stronger than that immediately after production of the internal olefin sulfonate composition.

Production Example A

(Synthesis of internal olefin having 16 carbon atoms)

Into a flask equipped with a stirrer were charged 7000 g (28.9 mol) of 1-hexadecanol "KALCOL 6098" (manufactured by Kao Corp.), and 700 g (10% by weight of the alcohol as the starting material) of γ-alumina (manufactured by STREM Chemicals, Inc.) as a solid acid catalyst. The alcohol was caused to react under stirring at 280°C for 3 hours and more while nitrogen (7000 mL/min.) was caused to flow into the system. After the end of the reaction, the conversion rate of the alcohol was 100%. The purity of the resultant C16 internal olefin was 99.6%. The resultant crude internal olefin was placed into a flask for distillation, and distilled at 136 to 160°C and 4.0 mmHg to yield an internal olefin having 16 carbon atoms and an olefin purity of 100%. The double bond distribution of the resultant internal olefin was as follows: C1 position: 2.3% by mass; C2 position: 23.6% by mass; C3 position: 18.9% by mass; C4 position: 17.6% by mass; C5 position: 13.6% by mass; C6 position: 11.4% by mass; and C7 and C8 positions: 7.4% by mass in total.

Production Example B

(Synthesis of internal olefin having 18 carbon atoms)

Into a flask equipped with a stirrer were charged 7000 g (25.9 mol) of 1-octadecanol "KALCOL 8098" (manufactured by Kao Corp.), and 700 g (10% by weight of the alcohol as the starting material) of γ-alumina (manufactured by STREM Chemicals, Inc.) as a solid acid catalyst. The alcohol was caused to react under stirring at 280°C for 10 hours and more while nitrogen (7000 mL/min.) was caused to flow into the system. After the end of the reaction, the conversion rate of the alcohol was 100%. The purity of the resultant C18 internal olefin was 98.2%. The resultant crude internal olefin was placed into a flask for distillation, and distilled at 148 to 158°C and 0.5 mmHg to yield an internal olefin having 18 carbon atoms and an olefin purity of 100%. The double bond distribution of the resultant internal olefin was as follows: C1 position: 2.2% by mass; C2 position: 27.7% by mass; C3 position: 20.7% by mass; C4 position: 15.5% by mass; C5 position: 10.6% by mass; C6 position: 8.5% by mass; C7 position: 6.6% by mass; and C8 and C9 positions: 4.1% by mass in total.

Production Example 1

(Method for producing internal olefin sulfonate (C16IOS))

The internal olefin having 16 carbon atoms produced in Production Example A was put into a thin film type sulfonation reactor having a jacket on the outside, and sulfur trioxide gas was supplied under the condition that cooling water at 20°C was passed through the outer jacket of the reactor, thereby to perform a sulfonation reaction. The molar ratio of SO$_3$/internal olefin during the sulfonation reaction was set to 1.005. The obtained sulfonated product was mixed with an aqueous alkali solution prepared with potassium hydroxide (alkaline agent) in an amount of 1.05 mole times the theoretical acid value, and then neutralized at 30°C for 1 hour by a continuous method. The neutralized product was hydrolyzed by heating in an autoclave at 170°C for 1 hour to obtain potassium internal olefin sulfonate having 16 carbon atoms. The content of the internal olefin sulfonate in which the sulfonic acid group is present at the 2-position in the obtained potassium internal olefin sulfonate was 14.9%, the content of the olefin sulfonate having a double bond at the terminal of the carbon chain was 1.1%, and the mass ratio of hydroxy form (potassium hydroxyalkane sulfonate)/olefin form (potassium olefin sulfonate) was 84/16. Further, the content of the raw material internal olefin contained in the obtained potassium internal olefin sulfonate was 0.5%, and the content of the inorganic compound was 1.2%.

Production Example 2

(Method for producing internal olefin sulfonate (C18IOS))

Potassium internal olefin sulfonate having 18 carbon atoms was obtained under the same conditions as in Production Example 1 except that the internal olefin having 18 carbon atoms produced in Production Example B was used. The content of the internal olefin sulfonate in which the sulfonic acid group is present at the 2-position in the obtained potassium internal olefin sulfonate was 17.7%, the content of the olefin sulfonate having a double bond at the terminal of the carbon chain was 1.0%, and the mass ratio of hydroxy form/olefin form was 80/20. Further, the content of the raw material internal olefin contained in the obtained potassium internal olefin sulfonate was 1.0%, and the content of the inorganic compound was 1.2%.

Production Example 3

(Method for producing internal olefin sulfonate (C16/C18IOS))

A sodium internal olefin sulfonate having a mass ratio (80/20) of 16/18 carbon atoms was obtained under the same conditions as in Production Example 1 except that a C16/C18 internal olefin prepared by mixing the internal olefin having 16 carbon atoms produced in Production Example A with the internal olefin having 18 carbon atoms produced in Production Example B at a mass ratio of 80/20 was used and an alkaline aqueous solution prepared with sodium hydroxide (alkaline agent) was used as a neutralizing agent. The content of the internal olefin sulfonate having a sulfonic acid group at the 2-position in the obtained sodium internal olefin sulfonate was 15.5%, the content of the olefin sulfonate having a double bond at the terminal of the carbon chain was 1.1%, and the mass ratio of hydroxy form/olefin form was 85/15. In addition, the content of the raw material internal olefin contained in the obtained sodium internal olefin sulfonate was 1.2%, and the content of the inorganic compound was 1.4%.
The internal olefin sulfonates obtained in Production Examples 1 to 3 were used after being concentrated if necessary.

Examples 1 to 4 and Comparative Examples 1 and 2

The C16IOS containing the alkaline agent, obtained in Production Example 1, was mixed with water (ion-exchanged water), and 0.15 parts by mass of sodium carbonate was added as a buffer to 100 parts by mass of the internal olefin sulfonate. An appropriate amount of citric acid was added to adjust the content of the alkaline agent and pH to prepare internal olefin sulfonate compositions each having the composition shown in Table 1.

Examples 5 and 6 and Comparative Examples 3 and 4

The C18IOS containing the alkaline agent, obtained in Production Example 2, was mixed with water (ion-exchanged water), and 0.15 parts by mass of sodium carbonate was added as a buffer to 100 parts by mass of the internal olefin sulfonate. An appropriate amount of citric acid was added to adjust the content of the alkaline agent and pH to prepare internal olefin sulfonate compositions each having the composition shown in Table 1.

Examples 7 to 9 and Comparative Examples 5 to7

The C16/C18IOS containing the alkaline agent, obtained in Production Example 3, was mixed with water (ion-exchanged water), and 0.15 parts by mass of sodium carbonate was added as a buffer to 100 parts by mass of the internal olefin sulfonate. An appropriate amount of citric acid was added to adjust the content of the alkaline agent and pH to prepare internal olefin sulfonate compositions each having the composition shown in Table 1.

[Table 1]

| | | C16/C18=100/0 | | | | | | C16/C18=0/100 | | | | C16/C18=80/20 | | | | | |
| | | Example 1 | Example 2 | Example 3 | Example 4 | Comparative Example 1 | Comparative Example 2 | Example 5 | Example 6 | Comparative Example 3 | Comparative Example 4 | Example 7 | Example 8 | Example 9 | Comparative Example 5 | Comparative Example 6 | Comparative Example 7 |
|---|---|---|---|---|---|---|---|---|---|---|---|---|---|---|---|---|---|
| Composition | Composition (% by mass) | | | | | | | | | | | | | | | | |
| | Internal olefin sulfonate | 59 | 59 | 59 | 59 | 59 | 59 | 51 | 51 | 51 | 51 | 66 | 66 | 33 | 66 | 66 | 66 |
| | Alkaline agent (KOH or NaOH) | 0.002 | 0.02 | 0.09 | 0.13 | 0 | 0.42 | 0.01 | 0.07 | 0.0007 | 0.21 | 0.07 | 0.10 | 0.07 | 0 | 0.001 | 0.34 |
| | Sodium carbonate | 0.09 | 0.09 | 0.09 | 0.09 | 0.09 | 0.09 | 0.08 | 0.08 | 0.08 | 0.08 | 0.1 | 0.1 | 0.05 | 0.1 | 0.1 | 0.1 |
| | Citric acid | Adjustment amount | Adjustment amount | Adjustment amount | Adjustment amount | Adjustment amount | Adjustment amount | Adjustment amount | Adjustment amount | Adjustment amount | Adjustment amount | Adjustment amount | Adjustment amount | Adjustment amount | Adjustment amount | Adjustment amount | Adjustment amount |
| | Water | Balance | Balance | Balance | Balance | Balance | Balance | Balance | Balance | Balance | Balance | Balance | Balance | Balance | Balance | Balance | Balance |
| | Total | 100 | 100 | 100 | 100 | 100 | 100 | 100 | 100 | 100 | 100 | 100 | 100 | 100 | 100 | 100 | 100 |
| | Content of alkaline agent (mmol) to internal olefin sulfonate 1 mol | 0.2 | 2 | 10 | 15 | 0 | 45 | 2 | 10 | 0.09 | 28 | 6 | 9.7 | 14 | 0 | 0.1 | 33 |
| Evaluation | pH at 25°C | 8.6 | 9.0 | 10.5 | 10.8 | 5.9 | 11.8 | 9.0 | 10.5 | 7.7 | 11.6 | 10.0 | 10.5 | 11.0 | 7.0 | 8.0 | 11.7 |
| | Hue change rate (%) | 5.0 | 3.0 | 1.9 | 4.9 | 67.5 | 8.5 | 3.2 | 1.6 | 22.2 | 11.1 | 1.3 | 1.3 | 4.9 | 28.4 | 8.3 | 14.0 |
| | Smell level | 2 | 1 | 1 | 1 | 5 | 4 | 1 | 1 | 5 | 4 | 1 | 1 | 2 | 5 | 4 | 4 |

As is clear from Table 1, it is found that the internal olefin sulfonate compositions of Examples 1 to 9 in which the content of the alkaline agent is 0.2 to 15 mmol per 1 mol of the internal olefin sulfonate has a small value of the hue change rate and the smell level, does not easily change the hue and smell even when preserved for a long period of time, and is excellent in stability, compared with the internal olefin sulfonate compositions of Comparative Examples 1 to 7 in which the content of the alkaline agent is out of the above range. The internal olefin sulfonate compositions of Examples 1 to 9 whose pH at 25°C is 8.5 or more and 11.0 or less have a smaller value of the hue change rate and the smell level, do not change easily the hue and the smell even when stored for a long period of time, and have excellent stability, compared to the internal olefin sulfonate compositions of Comparative Examples 1 to 7 in which the pH at 25°C is out of the above range.

INDUSTRIAL APPLICABILITY

The internal olefin sulfonate composition of the present invention is useful as a base for various detergents.

**Claims**

1. An internal olefin sulfonate composition comprising an internal olefin sulfonate and water, wherein
   when a content of the internal olefin sulfonate is 10% by mass or less, pH at 25°C is 8.5 or more and 11.0 or less, or
   when a content of the internal olefin sulfonate is more than 10% by mass, pH at 25°C when the content of the internal olefin sulfonate is adjusted with water to 10% by mass is 8.5 or more and 11.0 or less.

2. An internal olefin sulfonate composition comprising an internal olefin sulfonate, one or more kinds of alkaline agents selected from among an alkali metal hydroxide and an alkaline earth metal hydroxide, and water, wherein
   a content of the alkaline agent is 0.2 mmol or more and 15 mmol or less per 1 mol of the internal olefin sulfonate.

3. The internal olefin sulfonate composition according to claim 1 or 2, further comprising a buffer.

4. The internal olefin sulfonate composition according to claim 3, wherein a content of the buffer is 0.01 parts by mass or more and 3.0 parts by mass or less per 100 parts by mass of the internal olefin sulfonate.

5. The internal olefin sulfonate composition according to any one of claims 1 to 4, wherein an acid is further added.

6. The internal olefin sulfonate composition according to any one of claims 1 to 5, wherein the content of the internal olefin sulfonate in the internal olefin sulfonate composition is 10% by mass or more and 80% by mass or less.

7. The internal olefin sulfonate composition according to any one of claims 1 to 6, wherein the internal olefin sulfonate has 10 to 24 carbon atoms.

8. A method for preserving an internal olefin sulfonate in a composition comprising the internal olefin sulfonate and water, the method comprising:

   when a content of the internal olefin sulfonate in the composition is 10% by mass or less, adjusting pH at 25°C of the composition to 8.5 or more and 11.0 or less, or
   when a content of the internal olefin sulfonate in the composition is more than 10% by mass, adjusting pH at 25°C of a diluted solution to 8.5 or more and 11.0 or less, obtained by diluting the composition with water so that the content of the internal olefin sulfonate becomes 10% by mass.

9. A method for preserving an internal olefin sulfonate, comprising mixing an internal olefin sulfonate with at least one or more alkaline agents selected from among an alkali metal hydroxide and an alkaline earth metal hydroxide to form a composition,
   wherein a content of the alkaline agent in the composition is 0.2 mmol or more and 15 mmol or less per 1 mol of the internal olefin sulfonate.

10. The method for preserving an internal olefin sulfonate according to claim 8 or 9, wherein a buffer is further mixed in the composition.

11. The method for preserving an internal olefin sulfonate according to claim 10, wherein the content of the buffer in the composition is 0.01 parts by mass or more and 3.0 parts by mass or less per 100 parts by mass of the internal olefin sulfonate.

12. The method for preserving an internal olefin sulfonate according to any one of claims 8 to 11, wherein an acid is further mixed in the composition.

13. The method for preserving an internal olefin sulfonate according to any one of claims 8 to 12, wherein a content of the internal olefin sulfonate in the composition is 10% by mass or more and 80% by mass or less.

14. The method for preserving an internal olefin sulfonate according to any one of claims 8 to 13, wherein the internal olefin sulfonate has 10 to 24 carbon atoms.

**EP 3 719 103 A1**

<table>
<tr><td colspan="2" align="center">INTERNATIONAL SEARCH REPORT</td><td colspan="2">International application No.<br>PCT/JP2018/038284</td></tr>
</table>

A. CLASSIFICATION OF SUBJECT MATTER

Int.Cl. C11D1/14(2006.01)i, A61K8/46(2006.01)i, A61Q5/02(2006.01)i, A61Q19/10(2006.01)i, C07C303/42(2006.01)i, C07C309/20(2006.01)i

According to International Patent Classification (IPC) or to both national classification and IPC

B. FIELDS SEARCHED

Minimum documentation searched (classification system followed by classification symbols)
Int.Cl. C11D1/14, A61K8/46, A61Q5/02, A61Q19/10, C07C303/42, C07C309/20

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 3-126793 A (UNILEVER NV) 29 May 1991, page 7, lower right column, lines 1-4, table 2, page 5, lower right column, first paragraph, second paragraph<br>& GB 2236538 A, page 3, lines 2-5, page 9, lines 1-19, page 11, lines 18-24, page 14, lines 1-7, table 2 & CA 2026746 A1 & KR 10-1995-0005380 B | 1-14 |
| X | EP 0377261 A2 (SHELL INTERNATIONALE RESEARCH MAATSCHAPPIJ B.V.) 11 July 1990, formulation no. 11, page 4, line 5, examples<br>& US 5078916 A & GB 8900023 A | 1-14 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>05.11.2018 | Date of mailing of the international search report<br>20.11.2018 |
|---|---|
| Name and mailing address of the ISA/<br>　Japan Patent Office<br>　3-4-3, Kasumigaseki, Chiyoda-ku,<br>　Tokyo 100-8915, Japan | Authorized officer<br><br>Telephone No. |

Form PCT/ISA/210 (second sheet) (January 2015)

EP 3 719 103 A1

**INTERNATIONAL SEARCH REPORT**

International application No.

PCT/JP2018/038284

C (Continuation).    DOCUMENTS CONSIDERED TO BE RELEVANT

| Category* | Citation of document, with indication, where appropriate, of the relevant passages | Relevant to claim No. |
|---|---|---|
| X | JP 49-41179 B1 (LION FAT AND OIL CO., LTD.) 07 November 1974, example 1, column 6, line 28, column 14, lines 25, 26 (Family: none) | 1-14 |
| A | JP 7-507818 A (THE PROCTER & GAMBLE COMPANY) 31 August 1995, page 5, lower left column & JP 2003-147390 A & US 5529722 A, column 5, lines 15-25 & WO 1993/018123 A1 & EP 560001 A1 | 1-14 |
| A | JP 2012-007155 A (KAO CORPORATION) 12 January 2012, examples (Family: none) | 1-14 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014076983 A
- JP 2015178548 A
- JP 2016147928 A
- WO 2011052732 A

**Non-patent literature cited in the description**

- *J. Am. Oil Chem. Soc.,* 1992, vol. 69, 39